Europäisches Patentamt

European Patent Office

Office européen des brevets

(19)

(11) Publication number: **0 392 487**
**A2**

# EUROPEAN PATENT APPLICATION

(21) Application number: **90106918.7**

(22) Date of filing: **11.04.90**

(51) Int. Cl.⁵: **A61K 31/71, A61K 47/48**

(30) Priority: **13.04.89 JP 94091/89**

(43) Date of publication of application:
**17.10.90 Bulletin 90/42**

(84) Designated Contracting States:
**AT BE CH DE DK FR GB IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Kamei, Shigeru**
**1-509, 7 Sumiregaoka 1-chome, Takarazuka**
**Hyogo 665(JP)**
Inventor: **Yoshioka, Toshio**
**1-702, Takahatacho 1-chome, Nishinomiya**
**Hyogo 663(JP)**
Inventor: **Okada, Hiroaki**
**11-704, 44 Yamadaminami, Suita**
**Osaka 565(JP)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) Stabilized composition of anthracyclines.

(57) The present invention relates to a pharmaceutical composition containing an anthracycline antibiotic having an amino sugar residue and a water-soluble polyanion capable of at least 40% interacting with said antibiotic.

In the composition according to the invention, the anthracycline antibiotic is stabilized. The composition can be administered in various dosage forms.

EP 0 392 487 A2

## Stabilized Composition of Anthracyclines

TECHNICAL FIELD

This invention relates to a pharmaceutical composition which contains an anthracycline antibiotic stabilized therein.

BACKGROUND TECHNOLOGY

Amino sugar residue-containing anthracycline antibiotics are in general use as therapeutic agents for malignant tumors (anticancer agents) [of. e.g. Koseibusshitsu Taiyo (Compendium of Antibiotics) (3rd edition, enlarged), pp. 156-165, Tokyo Daigaku Shuppankai (University of Tokyo Press), 1984]. However, they should be used in limited doses because of their cumulative cardiotoxicity. Furthermore, when these anthracycline antibiotics are administered into the blood vessel, they disappear rapidly from the blood since they have small molecular weights; thus they have a reduced chance of being taken up by tumor cells. To solve this problem, various preparations have been proposed which contain an anthracycline antibiotic included in microspheres, microcapsules or liposomes and which can release the drug over 1 to 2 days, a week, a month or several months. However, the stability, in such preparations and in the living body, of anthracycline antibiotics included in such sacs or vesicles is not always satisfactory. While few reports are available on detailed investigations, reductions in drug efficacy due to degradation can reasonably be anticipated.

Japanese Patent Application Laid Open No. 63-215633 discloses that doxorubicin or $4'$-epi-doxorubicin in solution can be stabilized by adjusting the pH of the solution to 2.5 to 4.0. However, it is difficult to ensure pH control in various types of controlled release preparations over a long period of time. Furthermore, since said antibiotics are administered to the living body, it is desired that stabilization be made at a pH close to the physiological pH of the body.

For the administration of anthracycline antibiotics in the form of microsphere preparations, microcapsule preparations or liposome preparations to the living body, these preparations, in which many factors are dependent on interactions with the intrinsic functions of the living body, should meet multiple requirements. Since the matter is concerned with pharmaceuticals, it is required that preparations capable of meeting these variegated requirements as far as possible be provided.

When the above circumstances are taken into due consideration, the known anthracycline antibiotic preparations do not assure the stability of the anthracycline antibiotics included therein; it can hardly be said that satisfactory effects have been attained technologically.

For instance, in the case of the microsphere (microcapsule) preparation from which doxorubicin is released continuously over a month or so, the release data so far reported are values determined by measuring the fluorescence ascribable to the sum total of doxorubicin itself and doxorubicin-derived substances; they do not necessarily indicate that unchanged doxorubicin can be released continuously. In other words, continuous release of the drug in effective form may not be attained over the period during which such release is requied. Similarly, doxorubicin may be decomposed during storage of solutions of the doxorubicin preparation, resulting in a marked reduction in its quantity as compared with the initial value. In certain instances, the use of such a preparation as a drug encounters difficulties.

OBJECTS OF THE INVENTION

Under these circumstances, the inventors of the present invention made intensive investigations in an attempt to stabilize amino sugar residue-containing anthracycline antibiotics and, as a result, found that the stability of said anthracycline antibiotics can be increased by adding a certain kind of water-soluble polyanion to the anthracycline antibiotics to thereby cause formation of ion complexes. Further research works based on this finding have now led to completion of the present invention.

SUMMARY OF THE INVENTION

2

The invention relates to a composition which contains an anthracycline antibiotic having an amino sugar residue and a water-soluble polyanion capable of at least 40% interacting with said antibiotic.

DETAILED DESCRIPTION OF THE INVENTION

The amino sugar residue-containing anthracycline antibiotic to be used in the practice of the invention has a positively charged basic amino sugar moiety on the anthracycline skeleton.

As examples of said anthracycline antibiotic, there may be mentioned doxorubicin (adriamycin), 4'-epi-doxorubicin, 4'-desoxy-doxorubicin, 4'-desoxy-4'-iodo-doxorubicin, daunorubicin (daunomycin), 4-demethoxy-daunorubicin and aclarubicin.

Said anthracycline antibiotic may be in the form of a salt. Examples of the salt are physiologically acceptable salts with inorganic acids, such as hydrochloric acid, hydrobromic acid, sulfuric acid, phosphoric acid and nitric acid, or with organic acids, such as succinic acid, tartaric acid, ascorbic acid, citric acid, glutamic acid, benzoic acid, methanesulfonic acid and ethanesulfonic acid.

The water-soluble polyanion to be used in the practice of the invention includes natural and synthetic polymers having negatively charged acidic residues, such as sulfuric or sulfonic acid residues, and having a molecular weight of 500 to 2,000,000.

As the natural polymers, there may be mentioned polysaccharides preferably having 1.5 to 3 sulfuric acid residues per repeating unit sugar. The synthetic polymers include, among others, polystyrene and polyvinyl alcohol each preferably having 0.2 to 1.0 sulfuric acid residue per monomer unit.

Examples of the natural macromolecular polyanion are dextransulfate, ketaransulfate, heparansulfate, pullulansulfate, and sulfuric acid esters of fucoidan, heparin and curdlan and of partial hydrolyzates of these (cf. EP-A-0 351 809).

Said polyanion is preferably used in the form of a salt. Examples of the salt are physiologically acceptable inorganic salts, such as sodium, potassium, lithium, ammonium and calcium salts.

The interaction of polyanion with anthracycline antibiotic is expressed in terms of the percentage of the weight of anthracycline antibiotic with which a unit weight of polyanion can form an ion complex (cf. Example 1). In the practice of the invention, an interaction of 55-80% is particularly preferred.

The polyanions mentioned above may be used either singly or in the form of a mixture of two or more of them. They are used in an amount which may vary depending on the combination thereof with the anthracycline antibiotic. Generally, they are used in an amount of 1/100 to 1,000 parts by weight, preferably 1/50 to 200 parts by weight, more preferably 1/10 to 10 parts by weight, per part by weight of the anthracycline antibiotic.

The composition according to the invention may further contain an organic acid generally used for pH adjustment, such as citric acid, phosphoric acid or acetic acid, or a salt thereof. acetic acid, or a salt thereof.

The composition according to the invention can be produced, for example, by dissolving the anthracycline antibiotic in a buffer which is nearly neutral (pH 6-8), followed by addition of the polyanion to the solution.

The composition according to the invention can be administered as such to patients in the manner of injection, for example intramuscularly, subcutaneously, intravascularly, into organs or into tumor tissues or the like foci. The composition may be administered in the form of various controlled release preparations or targeting preparations. Thus the composition can be used as a starting material in the manufacture of such preparations.

Microsphere preparations, microcapsule preparations or liposome preparations which contain the composition according to the invention as included in respective sacs or vesicles can be produced by a per se known method using, among others, various natural or synthetic polymer or various phospholipids, which are in general use, for example those mentioned below. Thus, as such polymer, there may be mentioned gelatin, collagen, albumin, hemoglobin, transferrin, globulin, fibrin, dextran, pullulan, chitosan, mannan, cararageenan, amylopectin, pectin, lentinan, poly-fatty acid esters (homopolymers and copolymers of lactic acid, glycolic acid, malic acid, citric acid, etc.), poly-$\beta$-hydroxylactic acid, polyamino acids (poly-$\gamma$-benzyl-L-glutamic acid, poly-$\gamma$-methyl-L-glutamic acid, etc, polyaldehydes, polyvinylic polymers (homopolymers and copolymers of ethylene, propylene, butadiene, acrylic acid, acrylic acid esters, methacrylic acid, methacrylic acid esters, vinyl acetate, vinyl chloride, vinyl alcohol, vinylpyrrolidone, vinyl ethers, vinylcarbazole, styrene, styrene derivatives, $\alpha$-cyanoacrylic acid esters, acrylamide, divinylbenzene, etc.), maleic anhydride-based copolymers, and cellulose ethers (methylcellulose, ethylcellulose, carboxymethylcellulose, carboxyethylcellulose, hydroxyethylcellulose, and hydroxypropylcellulose, etc.), among others. As the

3

phospholipids, there may be mentioned, for example, phosphatidylcholine, phosphatidylethanolamine, phosphatidylserine, sphingomyelin, dicetyl phosphate, phosphatidylglycerol, phosphatidic acid and phosphatidylinositol.

Any of those preservatives, stabilizers, isotonizing agents, solubilizers and other ingredients which are generally used in injectable compositions may also be added to the composition according to the invention.

The composition according to the invention may further be used in producing other dosage forms than injections, for example mucosal preparations to be applied to the nasal, oral, rectal, vaginal, uterine mucosa or the like, transdermal preparations, or implantation preparations to be directly applied to tumor sites or sites of tumorectomy.

The composition according to the invnetion has the following characteristic features, among others:

(1) It assures good stability even in the neutral pH region in which anthracycline antibiotics are unstable and otherwise undergo decomposition (degradation); it can give preparations less irritant to the living tissue as compared with the conventional solutions of anthracycline antibiotics in which stability is secured by adjusting the pH to an extremely acidic level.

(2) The reaction or change of anthracycline antibiotics during the processes of making pharmaceuticals can be retained.

(3) Because of improved stability in the neutral pH region, the composition makes it possible to produce various controlled release preparations and targeting preparations which contain anthracycline antibiotics; in cases where long-term medication is required, such preparations, when administered weekly or monthly, can produce the desired pharmacological effects in a more stable manner.

Examples

The following examples illustrate the invention in further detail.

Example 1

The polyanions specified below in Table 1 were used. For each polyanion, two samples were prepared by adding 10 mg of the polyanion to 2 ml of a buffer solution (pH 6.86) with 0.2 mg of doxorubicin (hereinafter referred to as "DOX") hydrochloride dissolved therein. The samples were stirred at room temperature for 1 hour. Then, 2 ml of 2.5 M sodium chloride in buffer (pH 6.86) was added to one sample for causing dissociation of DOX from the ion complex formed by the preceding treatment. The other sample was deprived of the ion complex by means of a filter having a pore size of 0.45 μm. Both the samples thus treated were assayed for DOX. The quantity of DOX that had interacted ionically with the polyanion was estimated by calculating the difference between the quantity of DOX remaining in the fomer sample and the quantity of DOX remaining in the latter sample (filtrate).

For each polyanion, 10 mg of the polyanion was added to 2 ml of a buffer solution (pH 6.86) with 0.2 mg of DOX dissolved therein. The resulting mixture was stored at 37°C with stirring for 7 days. Then, 2 ml of 2.5 M sodium chloride in buffer (pH 6.86) was added to the mixture for causing dissociation of DOX from the ion complex that had formed. The remaining DOX was then assayed by the fluorescent HPLC method. The degree of stabilization was estimated by calculating the difference between the remaining DOX quantity thus found and the remaining DOX quantity found without using the polyanion and expressing the difference in percentage.

The DOX quantities (%) that reacted ionically with the respective polyanions and the DOX quantities (%) stabilized thereby are shown in Table 1.

Table 1

| Polyanion (mean molecular weight; salt) | Interaction (%) | Stabilization (%) |
|---|---|---|
| No addition | - | - |
| DS ( 5,000; Na) | 68.1 | 49.1 |
| DS ( 8,000; Na) | 67.5 | 58.3 |
| DS (500,000; Na) | 69.7 | 58.4 |
| Hep (Li) | 74.2 | 69.0 |
| Hep (NH$_4$) | 73.7 | 64.1 |
| Hep (Ca) | 75.2 | 67.4 |
| DS: Dextransulfate. Hep: Heparin. | | |

The interactions of some polyanions with daunorubicin hydrochloride and the quantities of daunorubicin stabilized thereby were examined in the same manner as above.

The daunorubicin quantities (%) that reacted ionically with the respective polyanions and the daunorubicin quantities (%) stabilized thereby are shown in Table 2.

Table 2

| Polyanion (salt) | Interaction (%) | Stabilization (%) |
|---|---|---|
| No addition | - | 0 |
| PVS (K) | 65.9 | 41.3 |
| Hep (Na) | 58.4 | 26.7 |
| Hep (CA) | 57.8 | 26.6 |
| PVS: Polyvinyl sulfate (mean molecular weight: 240,000). Hep: Heparin. | | |

Example 2

To 2 ml of a buffer solution (pH 6.86) with 0.2 mg of doxorubicin hydrochloride dissolved therein, there was added 50 μg or 300 μg of dextransulfate sodium (hereinafter referred to as "DS-Na") having a mean molecular weight of 5,000 or heparin sodium (hereinafter referred to as "Hep-Na"). The resulting mixture was stored at 37° C with stirring for 6 hours. Then, 2 ml of 2.5 M sodium chloride in buffer (pH 6.86) was added to the mixture for causing dissociation of DOX from the ion complex that had formed. The remaining DOX was assayed by the fluorescent HPLC method. The results obtained in this manner are shown in Table 3. The residual DOX percent shown for each case is a relative value with the residual DOX quantity found in a control run being taken as 100%. In said control run, a buffer solution having a pH of 4.01 was used in lieu of the above-mentioned buffer solution (pH 6.86) and 2.5 M sodium chloride in buffer (pH 6.86) and the storage temperature was 0° C.

Table 3

| Polyanion | Addition level (Note a) | Residual DOX (%) after 6 hours (Note b) |
|---|---|---|
| No addition | - | 38.4 |
| DS-Na | 25 | 67.2 |
| DS-Na | 100 | 48.9 |
| Hep-Na | 25 | 75.4 |
| Hep-Na | 100 | 79.5 |
| Notes: | | |
| a) Polyanion quantity (% by weight) relative to DOX. b) Buffer pH 6.86, 37° C. | | |

Without addition of any polyanion, DOX was decomposed rapidly and its quantity decreased to 38.4% in 6 hours. On the contrary, when each polyanion was used at addition levels of 25% and 100%, the residual DOX percentage after 6 hours of storage increased with the addition level and, in the case of DS-Na and Hep-Na, the decomposition of DOX was only about one third as compared with the case where no polyanion was added.

Example 3

To 2 ml of a buffer solution (pH 6.86) with 0.1 mg of doxorubicin hydrochloride dissolved therien, there was added 10 mg of one of three DS-Na species differing in mean molecular weight. Each resultant mixture was stored at 37° C with stirring for 7 days and the residual DOX was then assayed in the same manner as in Example 2. The results obtained in this manner are shown in Table 4. The respective residual DOX data are relative values calculated in the same manner as in Example 2.

Table 4

| Mean molecular weight of DS-Na | DS-Na addition level (Note a) | Residual DOX (%) after 7 days of storage (Note b) |
|---|---|---|
| No addition | - | 2.4 |
| 5,000 | 100 | 51.5 |
| 8,000 | 100 | 60.7 |
| 500,000 | 100 | 60.8 |
| Notes: | | |
| a) Polyanion quantity (% by weight) relative to DOX. b) Buffer pH 6.86, 37° C. | | |

Without addition of DS-Na, the DOX quantity decreased to 2.4% in 7 days. On the contrary, when DS-Na species differing in molecular weight were respectively added, 50-60% of DOX could remain unchanged. The stabilizing effect showed a tendency toward increase with the increasing molecular weight of DS-Na.

Exampel 4

To 2 ml of a buffer solution (pH 6.86) with 0.1 mg of doxorubicin hydrochloride dissolved therein, there was added 10 mg of one of several heparin salts. The mixture was stored at 37° C with stirring for 7 days.

6

Thereafter, the residual DOX percentage was calculated in the same manner as in Example 2. The results obtained in this manner are shown in Table 5.

Table 5

| Heparin salt | Addition level | Residual DOX (%) after 7 days of storage (Note b) |
|---|---|---|
| No addition | - | 2.4 |
| Sodium | 100 | 78.8 |
| Lithium | 100 | 71.4 |
| Ammonium | 100 | 66.5 |
| Calcium | 100 . | 69.8 |
| Notes: | | |
| a) Polyanion quantity (% by weight) relative to DOX. b) Buffer pH 6.86, 37° C. | | |

Without addition of any heparin salt, the quantity of DOX decreased to 2.4% in 7 days. On the contrary, 66.5-78.8% of DOX remained unchanged in all cases where heparin salts were added. Although some differences were noted in stabilizing effect among the heparin salts used, the differences were not significant.

Example 5

Doxorubicin hydrochloride (20 mg) was added to 0.15 ml of water, followed by addition of 10 mg of DS-Na with a mean molecular weight of 5,000. The suspension thus obtained was added to a solution of 2 g of a lactic acid-glycolic acid copolymer (lactic acid/glycolic acid mole ratio = 75/25; weight average molecular weight = 10,500; hereinafter referred to as "PLGA" in 2.05 g of dichloromethane. Dispersion was effected for 30 seconds using a probe-type sonicator. The resulting emulsion was poured into 400 ml of a 0.1% aqueous solution of polyvinyl alcohol and the resulting mixture was treated with a small-size homogenizer to give a three-phase emulsion. The dichloromethane in this emulsion was allowed to evaporate under stirring and the resulting microspheres (hereinafter referred to as "msp") were collected by centrifugation. The msp were again dispersed in distilled water and once more centrifuged for elimination of that portion of the drug which was remaining in free from. The msp thus washed and recovered were lyophilized for more complete solvent removal and dehydration.

The msp (lot A-102) prepared in the above manner and the msp (lot A-101) prepared in the same manner but without adding DS-Na were submitted to a DOX release test. The results found after 7 days are shown in Table 6. In the release test, the msp were dispersed in a release test solution composed of 1/30 M phosphate buffer (pH 7.0) and 0.2% (by weight) heparin calcium salt.

7

Table 6

| Lot | Level of addition of DS-Na (Note a) | I (%) | II (%) | I + II (%) |
|---|---|---|---|---|
| A-101 | 0 | 45.9 | 30.0 | 75.9 |
| A-102 | 0.5 | 40.9 | 52.5 | 93.4 |
| Notes: | | | | |
| a) Weight ratio of DS-Na to DOX. I: Quantity of DOX in msp (% against the initial value after 7 days incubation). II: Quantity of DOX in release test solution (% against the initial value after 7 days incubation). | | | | |

The msp prepared with DS-Na added showed a higher rate of release as compared with the msp prepared without addition of DS-Na. However, the sum of the DOX quantity in msp and that in release test solution amounted to 93.4% with the former msp and the corresponding sum for the latter (without DS-Na was 75.9% and thus smaller by about 18%. Since the release test solution contained heparin calcium as a stabilizer, the above difference was presumably due to decomposition in msp. It was thus shown that the addition of DS-Na can improve the stability of DOX in msp in a significant manner.

Example 6

Doxorubicin (8 mg) was dissolved in 4 ml of water, 16 mg of DS-Na having a molecular weight of 8,000 was added to the solution, 1.5 g of alkali-treated gelatin was further added, and the whole mixture was heated to 70°C. To the warmed mixture were added 30 ml of sesame oil and 1 ml of sorbitan sesquioleate, each warmed to 70°C. The resultant mixture was converted to a water-in-oil emulsion using a small-size homogenizer. The emulsion was immediately cooled to 0°C and allowed to stand at that temperature for 20 minutes, followed by centrifugation. The supernatant oil layer was replaced with a saturated solution of glutaraldehyde (GA) in sesame oil and the resultant mixture was stirred at room temperature for 3 hours for effecting the crosslinking reaction. The emulsion was then centrifuged, and the supernatant was replaced with an equal volume of acetone. This washing procedure was repeated three times in all, followed by drying under reduced pressure, which gave DOX-containing gelatin msp.

The msp prepared in the above manner were thoroughly disintegrated with collagenase and the unchanged residual DOX that had been included in the msp was quantitated and the percent inclusion of DOX was calculated. The results thus obtained are shown in Table 7.

Table 7

| Lot | Amount of DS-Na (mg) | Crosslinking with GA | Percent inclusion (%) | Residual percentage (%) at GA crosslinking stage |
|---|---|---|---|---|
| G-101 | 0 | No | 69.3 | 100 |
| G-102 | 0 | Yes | 3.3 | 4.8 [a] |
| G-201 | 16 | No | 87.0 | 100 |
| G-202 | 16 | Yes | 32.8 | 42.3 [b] |

[a] % Inclusion in G-102/% inclusion in G-101.
[b] % Inclusion in G-202/% inclusion in G-201.

While the inclusion rate in G-101 was 69.3%, the inclusion rate in G-201 prepared with added DS-Na was 87.0%, namely higher by as much as 17.7%. This difference presumably resulted from the addition of DS-Na having produced an improvement in stability of DOX in the water-in-oil emulsion formation step under high temperature conditions (70°C).

Furthermore, the ratio of the DOX inclusion percentage in G-102 to that in G-101 was as low as 4.8%. On the contrary, the ratio of the DOX inclusion percentage in G-202 to that in G-201 was 42.3%, namely higher by 37.5%. This difference suggests that the addition of DS-Na protected DOX against its denaturation by means of the crosslinking agent in the GA crosslinking reaction step and against effects of additives and other factors.

The results of these experiments indicate that the addition of DS-Na can stabilize DOX to a sufficient extent for it to remain unchanged in such steps as heat treatment and crosslinking reaction steps for preparing more useful dosage forms thereof.

## Claims

1. A pharmaceutical composition which contains an anthracycline antibiotic having amino sugar residue and a water-soluble polyanion capable of at least 40% interacting with said antibiotic.

2. The composition as claimed in claim 1, wherein the anthracycline antibiotic is doxorubicin, 4′-epidoxorubicin, 4′-desoxy-doxorubicin, 4′-desoxy-4′-iodo-doxorubicin, daunorubicin, 4-demethoxy-daunorubicin or aclarubicin.

3. The composition as claimed in claim 2, wherein the anthracycline antibiotic is a member selected from the group consisting of doxorubicin and daunorubicin.

4. The composition as claimed in claim 1, wherein the anthracycline antibiotic forms a physiologically acceptable salt with inorganic acid or organic acid.

5. The composition as claimed in claim 1, wherein the water-soluble polyanion is a natural or synthetic polymer having negatively charged acidic residue and having a molecular weight of 500 to 2,000,000.

6. The composition as claimed in claim 5, wherein the natural polymer is a polysaccharide having 1.5 to 3 sulfuric acid or sulfonic acid residues per repeating unit sugar.

7. The composition as claimed in claim 6, wherein the natural polymer is dextransulfate, ketaransulfate, heparansulfate, pullulansulfate, sulfuric acid ester of fucoidan, heparin, curdlan or partial hydrolyzate of curdlan.

8. The composition as claimed in claim 7, wherein the natural polymer is a member selected from the group consisting of dextran sulfate, heparin, curdlan and partial hydrolyzate of curdlan.

9. The composition as claimed in claim 5, wherein the synthetic polymer is polystyrene or polyvinyl alcohol having 0.2 to 1.0 sulfuric acid residue per monomer unit.

10. The composition as claimed in claim 1, wherein the water-soluble polyanion forms a physiologically acceptable salt.

11. The composition as claimed in claim 1, wherein the amount of the water-soluble polyanion contained therein is 1/100 to 1,000 per part by weight of the anthracycline antibiotic.

12. The composition as claimed in claim 1, wherein the interaction of the polyanion with the anthracycline antibiotic is 55 to 80%.

13. The composition as claimed in claim 1, wherein the composition is further formulated to a controlled release preparation together with a polymer for microsphere.

14. A method for producing the composition as claimed in claim 1, which comprises dissolving the anthracycline antibiotic in a buffer which is nearly neutral, and followed by addition of the polyanion to the solution.